# EUROPEAN PATENT APPLICATION

(11) **EP 3 561 759 A1**
(43) Date of publication of application: **30.10.2019**
(21) Application number: 17882586.5
(22) Date of filing: 20.12.2017
(51) Int. Cl.: G06Q 50/04, C10G 45/72

(54) **SERVER, METHOD, AND PROGRAM FOR SUPPLYING DESULFURIZATION CATALYST-RELATED INFORMATION, AND COMPUTER-READABLE RECORDING MEDIUM RECORDING SAME**

(30) Priority: 21.12.2016 JP 2016247762
(71) Applicant: Cosmo Oil Co., Ltd., Tokyo 105-8528 (JP)
(72) Inventor: NAKAJIMA Nobumasa, Tokyo 105-8528 (JP); KIKUCHI Susumu, Tokyo 105-8528 (JP); IDEI Kazuo, Satte-shi Saitama 340-0193 (JP)
(74) Representative: Hoffmann Eitle
(86) International application number: PCT/JP2017/045733
(87) International publication number: WO 2018/117154

(57) **Abstract**

Provided are a desulfurization catalyst performance prediction system and a desulfurization catalyst performance prediction program with which the performance of a desulfurization catalyst is predicted with high accuracy, without performing a complicated process such as a plant simulation. A server, which is connected to a user terminal via a network, and which supplies a desulfurization catalyst lifetime of a user plant to a user on the basis of desulfurization catalyst bench plant data and a desulfurization catalyst lifetime function, is equipped with a processor and a storage means that stores computer-readable command. When a computer-readable command is executed by the processor, the server receives user plant-related data and a user desulfurization catalyst performance prediction condition from the user terminal to the user plant on the basis of a comparison between the desulfurization catalyst bench plant data and the obtained user plant-related data, calculates a catalyst lifetime for the user's desulfurization catalyst on the basis of the obtained user desulfurization catalyst performance prediction condition and the user desulfurization catalyst lifetime function, and transmits the calculated catalyst lifetime to the user terminal.

## Description

### Technical Field

The present invention relates to a server, a method, a program and a computer-readable recording medium storing the same, for supplying desulfurization catalyst-related information to a user.

### Background Art

In a petroleum refining process, a desulfurization has conventionally been performed to remove a sulfur content contained in a feedstock. For example, hydrodesulfurization in which a petroleum fraction is passed through a catalyst together with hydrogen under high temperature and high pressure to thereby remove impurities such as a sulfur content etc. from the petroleum fraction has been generally known as the desulfurization performed in a petroleum refinery and the like.

The catalyst used for such desulfurization is degraded in accordance with use. The degradation of the catalyst changes operating conditions of a desulfurization plants, such as required temperature and hydrogen consumption. For this reason, degradation of the catalyst with passage of time is simulated in advance to obtain most suitable operating conditions of the desulfurization plants (e.g., Patent Documents 1 to 4 and the like).

For example, Patent Document 1 discloses a catalyst use supporting method. In this method, information about catalyst reaction proceeding in a reactor operated by a chemical company (user) is transmitted to a catalyst supplying company via a communication network, and the catalyst supplying company generates operation information for the reactor by use of a simulator apparatus based on the information about the catalyst reaction, and sends the operation information back to the chemical company via the communication network.

Simulation is performed in this manner based on the information about the catalyst reaction. Accordingly, a lifetime of the catalyst can be predicted, and an operation, for example, for increasing/decreasing production can be performed according to circumstances. Therefore, the catalyst can be used efficiently so that production cost of a chemical product can be reduced.

### Prior Art Documents

### Patent Documents

Patent Document 1: JP2003-58206A
Patent Document 2: JPH10-60455A
Patent Document 3: JP2003-27069A
Patent Document 4: JP2007-126684A

### Summary of Invention

### Technical Problem

However, the method disclosed in Patent Document 1 is aimed at simulating a reaction state in a computer. To this end, for example, details of equipment specifications etc. related to the reaction, such as the number of tubes in the reactor and the length of each of the tubes have to be input to the computer. This input is however complicated.

In addition, in Patent Document 1, the degradation of the catalyst with passage of time is predicted in a manner originated by technology held by the catalyst supplying company. For example, a dynamic model using a catalyst reaction rate equation is used as a prediction engine. However, information for the catalyst lifetime prediction held by the catalyst supplying company provides prediction merely in an environment in which the catalyst supplying company has performed analysis. In some cases, the information for the catalyst lifetime prediction may show a large difference from a change in the performance of the catalyst in a commercial plant.

Particularly, product oil is refined from various feedstock oils in petroleum refining, differently from petrochemistry in which a product is manufactured from single feedstock oil. Therefore, the number of necessary parameters for the degradation prediction of the desulfurization catalyst is overwhelmingly large. For this reason, accuracy of performance simulation of the desulfurization catalyst for the desulfurization units provided in a petroleum refining plant etc. cannot be enhanced unless sufficient pilot plant data is held.

When there is a large difference between a simulation result and a change of the catalyst performance in a commercial plant, the catalyst cannot be efficiently used. Therefore, manufacturing cost of produced oil increases.

In consideration of such a situation, it is an object of the present invention to provide a server, a method, a program and a computer-readable recording medium storing the same, for performing highly accurate performance prediction of a desulfurization catalyst without performing a complicated process such as a plant simulation, and for easily supplying desulfurization catalyst-related information about the highly accurate performance prediction of the desulfurization catalyst to a user who does not have necessary information for the performance prediction of the desulfurization catalyst.

### Means for Solving the Problem

The present invention has been made to solve the aforementioned problem in the background art. A server according to the present invention supplies desulfurization catalyst-related information. The server is to be connected to a user terminal via a network to supply a desulfurization catalyst lifetime of a user plant to a user based on desulfurization catalyst pilot plant data and a desulfurization catalyst lifetime function. The server includes a processor and a memory storing computer-readable instructions. When the computer-readable instructions are executed by the processor, the server receives user plant-related data and a user desulfurization catalyst performance prediction condition from the user terminal, generates from the desulfurization catalyst lifetime function a user desulfurization catalyst lifetime function tailored to the user plant based on a comparison between the desulfurization catalyst pilot plant data and the received user plant-related data, calculates a catalyst lifetime for a user desulfurization catalyst based on the received user desulfurization catalyst performance prediction condition and the user desulfurization catalyst lifetime function, and transmits the calculated catalyst lifetime to the user terminal.

The server according to the present invention may be configured such that, when the computer-readable instructions are executed by the processor, the server further generates from a hydrogen consumption function a user hydrogen consumption function tailored to the user plant based on the comparison between the desulfurization catalyst pilot plant data and the received user plant-related data, calculates a hydrogen consumption for the user desulfurization catalyst based on the received user desulfurization catalyst performance prediction condition and the user hydrogen consumption function, and transmits the calculated hydrogen consumption to the user terminal.

The server according to the present invention may be configured such that, when the computer-readable instructions are executed by the processor, the server further generates from a product yield function a user product yield function tailored to the user plant based on the comparison between the desulfurization catalyst pilot plant data and the received user plant-related data, calculates a product yield for the user desulfurization catalyst based on the received user desulfurization catalyst performance prediction condition and the user product yield function, and transmits the calculated product yield to the user terminal.

The server according to the present invention may be configured to calculate a tuning parameter based on a comparison between the user plant-related data and the desulfurization catalyst pilot plant data, and to generate, from the tuning parameter and the desulfurization catalyst-related function, the user desulfurization catalyst lifetime function tailored to the user plant.

With the server according to the present invention, the user plant-related data may include at least one of user plant operating data, the user plant operating data being operating data for a predetermined period in the user plant and including at least an operating condition, a feedstock oil characteristic and a produced oil characteristic, a kind of catalyst to be used in the user plant, and equipment setting information of the user plant.

With the server according to the present invention, the user desulfurization catalyst performance prediction condition may include at least one of an operating condition, a feedstock oil characteristic and a produced oil characteristic.

With the server according to the present invention, the desulfurization catalyst lifetime function may include at least one of a degradation function for direct desulfurization, a degradation function for indirect desulfurization, a degradation function for light gas oil desulfurization, and a degradation function for kerosene desulfurization.

The server according to the present invention may be configured to receive the user plant-related data including at least a kind of catalyst and to generate the user desulfurization catalyst lifetime function based on the desulfurization catalyst pilot plant data corresponding to the kind of catalyst.

A method according to the present invention is executed by a processor to supply desulfurization catalyst-related information. The method supplies a desulfurization catalyst lifetime of a user plant to a user via a network based on desulfurization catalyst pilot plant data and a desulfurization catalyst lifetime function. The method includes steps of receiving user plant-related data and a user desulfurization catalyst performance prediction condition from the user terminal, generating from the desulfurization catalyst lifetime function a user desulfurization catalyst lifetime function tailored to the user plant based on a comparison between the desulfurization catalyst pilot plant data and the received user plant-related data, calculating a catalyst lifetime for a user desulfurization catalyst based on the received user desulfurization catalyst performance prediction condition and the user desulfurization catalyst lifetime function, and transmitting the calculated catalyst lifetime information to the user terminal.

The method according to the present invention may include steps of generating, from a hydrogen consumption function, a user hydrogen consumption function tailored to the user plant based on the comparison between the desulfurization catalyst pilot plant data and the received user plant-related data, calculating a hydrogen consumption for the user desulfurization catalyst based on the received user desulfurization catalyst performance prediction condition and the user hydrogen consumption function, and transmitting the calculated hydrogen consumption to the user terminal.

The method according to the present invention may include generating, from a product yield function, a user product yield function tailored to the user plant based on the comparison between the desulfurization catalyst pilot plant data and the received user plant-related data, calculating a product yield for the user desulfurization catalyst based on the received user desulfurization catalyst performance prediction condition and the user product yield function, and transmitting the calculated product yield to the user terminal.

The method according to the present invention may include steps of calculating a tuning parameter based on a comparison between the user plant-related data and the desulfurization catalyst pilot plant data, and generating, from the tuning parameter and the user desulfurization catalyst lifetime function, the user desulfurization catalyst lifetime function tailored to the user plant.

With the method according to the present invention, the user plant-related data may include at least one of user plant operating data, the user plant operating data being operating data for a predetermined period in the user plant and including at least an operating condition, a feedstock oil characteristic and a produced oil characteristic, a kind of catalyst to be used in the user plant, and equipment setting information of the user plant.

With the method according to the present invention, the desulfurization catalyst performance prediction condition may include at least one of an operating condition, a feedstock oil characteristic and a produced oil characteristic.

With the method according to the present invention, the desulfurization catalyst lifetime function may include at least one of a degradation function for direct desulfurization, a degradation function for indirect desulfurization, a degradation function for light oil desulfurization, and a degradation function for kerosene desulfurization.

With the method according to the present invention, at least a kind of catalyst may be received as the user plant-related data so that the user desulfurization catalyst lifetime function can be generated based on the desulfurization catalyst pilot plant data corresponding to the kind of catalyst.

### Advantageous Effect of Invention

According to the present invention, a user desulfurization catalyst-related function tailored to a user plant can be generated from a desulfurization catalyst-related function obtained in advance in a pilot plant for each catalyst, operating data of a user plant, etc. Thus, the performance of the desulfurization catalyst can be predicted with high accuracy without performing a complicated process such as a plant simulation.

Even when a user does not have necessary information for the performance prediction of the desulfurization catalyst, the performance of the desulfurization catalyst can be predicted with high accuracy by simply inputting certain information to a user terminal.

### Brief Description of Drawings

Fig. 1 is a systematic configuration view illustrating a schematic configuration of a desulfurization catalyst performance prediction system according to one example.
Fig. 2 is a flow chart illustrating a flow of generating a user desulfurization catalyst-related function using the desulfurization catalyst performance prediction system of Fig. 1.
Fig. 3 is a flow chart illustrating a flow of calculating desulfurization catalyst-related information using the desulfurization catalyst performance prediction system of Fig. 1.
Fig. 4 is an example of a display screen for inputting desulfurization catalyst performance prediction conditions from a user terminal.
Fig. 5 is an example of a display screen of the desulfurization catalyst-related information displayed on the user terminal.
Fig. 6 is another example of the display screen of the desulfurization catalyst-related information displayed on the user terminal.

### Embodiments of Invention

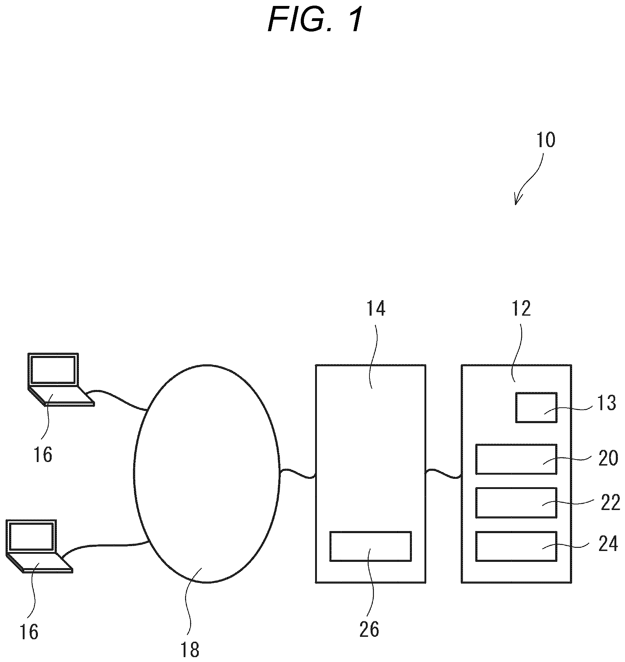

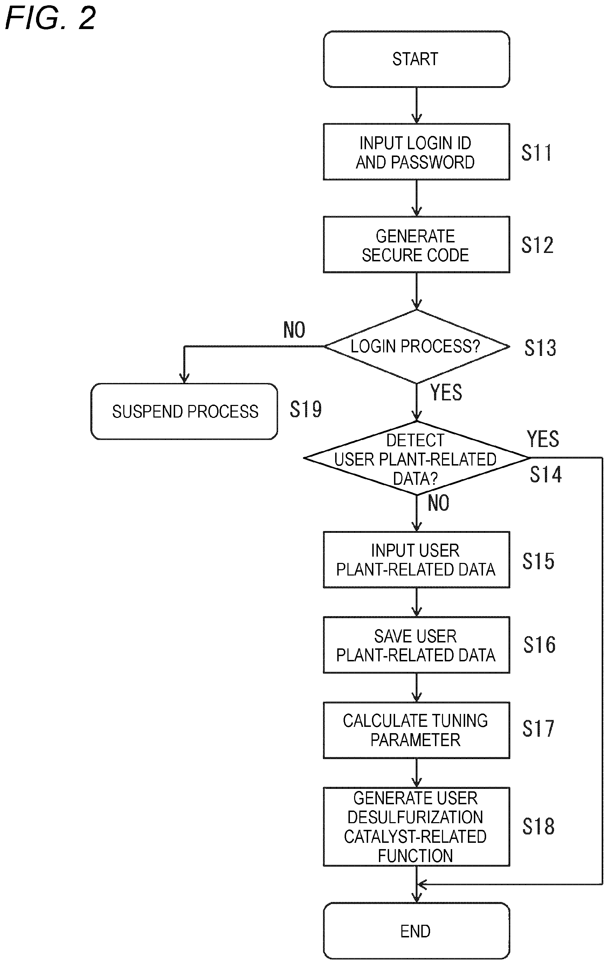

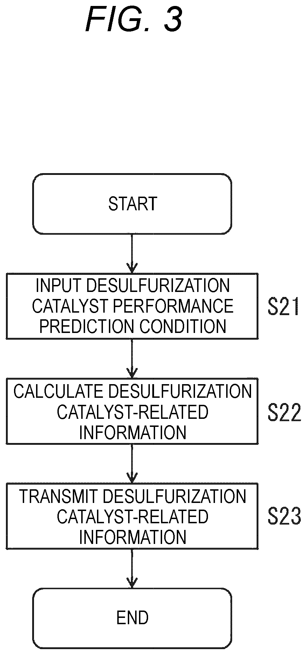

Hereinafter, embodiments (examples) of the present invention will be described in more detail with reference to the drawings. Fig. 1 is a systematic configuration view illustrating a schematic configuration of a desulfurization catalyst performance prediction system according to one example. Fig. 2 is a flow chart illustrating a flow of generating a user desulfurization catalyst-related function using the desulfurization catalyst performance prediction system of Fig. 1. Fig. 3 is a flow chart illustrating a flow of calculating desulfurization catalyst-related information using the desulfurization catalyst performance prediction system of Fig. 1.

As shown in Fig. 1, the desulfurization catalyst performance prediction system 10 according to this example has a server computer 12 and a communication server computer 14. The server computer 12 has a desulfurization catalyst pilot plant data memory 20, a user plant information memory 22, a desulfurization catalyst-related function memory 24, a processor 13, etc. The communication server computer 14 is connected to user terminals 16 via a network 18.

The server computer 12 and the communication server computer 14 may be any existing computer, and, for example, cloud computing may be used.

In this example, the server computer 12 for arithmetic processing and the communication server computer 14 for communication processing are configured as separate computers. Thus, the server computer 12 for arithmetic processing is protected from unauthorized access etc. It is a matter of course that the server computer 12 and the communication server computer 14 may be configured into one computer, or each of the server computer 12 and the communication server computer 14 may be constituted by a plurality of computers by use of distributed computing.

Desulfurization catalyst pilot plant data is stored for each kind of catalyst in the desulfurization catalyst pilot plant data memory 20. The "desulfurization catalyst pilot plant data" in the present description is data that is obtained by an experiment on a desulfurization catalyst by the pilot plant. For example, required temperature, a tendency to catalyst degradation (degradation factor), or the like is included in the "desulfurization catalyst pilot plant data".

User plant-related data is stored for each user in the user plant information memory 22. The "user plant-related data" in the present description is operating data for a predetermined period in a user plant owned by the user. At least one of operating data of the user plant, a kind of catalyst to be used in the user plant, and equipment setting information of the user plant is included in the "user plant-related data". The operating data includes at least operating conditions, feedstock oil characteristics and produced oil characteristics. When a user desulfurization catalyst-related function has been generated, the user desulfurization catalyst-related function is also stored as the user plant-related data in the user plant information memory 22, as will described later.

The "equipment setting information" in the present description is information about working conditions of the user plant. The "equipment setting information" includes, for example, an upper limit temperature for operation, an upper limit pressure for operation, an upper limit processing quantity for operation, a catalyst filling amount, etc., in the user plant.

A desulfurization catalyst-related function which is a model expression is stored in the desulfurization catalyst-related function memory 24. In the present description, the "desulfurization catalyst-related function" includes at least a desulfurization catalyst lifetime function for calculating a catalyst lifetime for the desulfurization catalyst. The "desulfurization catalyst-related function" means a function or a function group including a hydrogen consumption function for calculating hydrogen consumption or a product field function for calculating a product yield in addition to the desulfurization catalyst lifetime function.

In the present description, the "catalyst lifetime" is a concept including not only an original lifetime (a period between a use start and a use limit) of the desulfurization catalyst but also a period or day and time which can be calculated from the catalyst lifetime, such as a period between a present time point and the use limit, day and time of the use limit, recommended day and time which is not the use limit but the day and time at which exchange is recommended (for example, half a year before the use limit, etc.).

The server computer 12 according to the present example is configured to transmit the "catalyst lifetime" including the concept of the period or the day and time to one of the user terminals 16 based on the calculated lifetime of the desulfurization catalyst, as will be described later.

The desulfurization catalyst lifetime function expresses a degradation level of the desulfurization catalyst as a function of the number of days t of oil passage. The degradation level φ of the desulfurization catalyst is defined as a ratio of a reaction rate constant Kt for oil passage of t days to a reaction rate constant K0 for oil passage of 0 day. That is, the degradation level φ can be expressed as φ= Kt/K0.

The degradation of the desulfurization catalyst is mainly influenced by coke degradation and metal degradation. Therefore, it is preferable that each of a function expression based on the coke degradation and a function expression based on the metal degradation is assumed as the desulfurization catalyst lifetime function.

The coke degradation means catalyst degradation due to cokes generated in a catalyst reaction process. The metal degradation means catalyst degradation due to disposition of a metal content contained in feedstock oil.

For example, there are a plurality of methods such as direct desulfurization, indirect desulfurization, light gas oil desulfurization and kerosene desulfurization. Degradation rates of the desulfurization catalyst by the methods vary from one another. Accordingly, the desulfurization catalyst lifetime function varies from one of the methods to another. Therefore, it is preferable that at least one of a degradation function for the direct desulfurization, a degradation function for the indirect desulfurization, a degradation function for the light gas oil desulfurization and a degradation function for the kerosene desulfurization is stored as the desulfurization catalyst lifetime function in a catalyst degradation function memory.

The hydrogen consumption function or the product yield function can be calculated by a background-art existing method. The desulfurization catalyst pilot plant data memory 20, the user plant information memory 22, and the desulfurization catalyst-related function memory 24 may be formed by an internal recording medium such as a hard disk drive or a solid state drive of the server computer 12 or may be formed by an external recording medium such as an external hard disk drive or a USB memory connected to the server computer 12.

The communication server computer 14 has a user information memory 26 in which a user identification code of each user of the desulfurization catalyst performance prediction system 10 is stored. For example, a secure code uniquely determined by a combination of a login ID and a password can be used as the user identification code.

The network 18 can be any computer network without any specific limitation, and may be the Internet or an intranet. As for the user terminals 16, any terminals can be used, for example, a general-purpose computer such as a desktop computer, a laptop computer or a tablet computer, etc. or a dedicated computer used in the user plant.

The desulfurization catalyst performance prediction system 10 of the present example configured in this way generates a user desulfurization catalyst-related function by a flow shown in Fig. 2. The generation of the following user desulfurization catalyst-related function can be implemented as a program (computer-readable instructions) executed by the processor 13 of the server computer 12.

First, a user uses one of the user terminals 16 to make connection to the communication server computer 14. Thus, a login screen is displayed on the user terminal 16, and the user inputs a login ID and a password (S11).

The communication server computer 14 generates a secure code from the input login ID and the input password (S12) and matches the secure code with user identification codes stored in the user information memory 26 (S13). When there is one of the user identification codes matched with the secure code, the login succeeds so that the process is continued. When there is none of the user identification codes matched with the secure code, the login fails so that the process is suspended (S19).

When the login succeeds, searching is performed to find out whether user plant-related data of the logging-in user is present or not in the user plant information memory 22 of the server computer 12 (S14). When the user plant-related data is present in the user plant information memory 22, desulfurization catalyst-related information is calculated by use of a user desulfurization catalyst-related function stored in the user plant information memory 22, as will be described later.

On the other hand, when the user plant-related data of the logging-in user is not present in the user plant information memory 22, a screen for inputting the user plant-related data is displayed on the user terminal 16, and the user inputs the user plant-related data (S15).

Upon reception of the input user plant-related data, the server computer 12 stores the user plant-related data into the user plant information memory 22 (S16). The server computer 12 compares the user plant-related data stored in the user plant information memory 22 with desulfurization catalyst pilot plant data stored in the desulfurization catalyst pilot plant data memory 20 to thereby calculate tuning parameters (S17).

Here, the tuning parameters are parameters for correcting differences between the user plant-related data and the desulfurization catalyst pilot plant data. The differences between the user plant-related data and the desulfurization catalyst pilot plant data are, for example, a machine difference between the pilot plant and the user plant, a difference between operating conditions in the pilot plant and operating conditions in the user plant, a difference between a kind of oil generated in the pilot plant and a kind of oil generated in the user plant, etc.

A desulfurization catalyst-related function which is a model expression stored in the desulfurization catalyst-related function memory 24 is corrected based on the thus obtained tuning parameters so that a user desulfurization catalyst-related function tailored to the user plant is generated (S18).

Next, the desulfurization catalyst performance prediction system 10 calculates desulfurization catalyst-related information, as shown in Fig. 3. The calculation of the following desulfurization catalyst-related information can be implemented as a program (computer-readable instructions) executed by the processor 13 of the server computer 12.

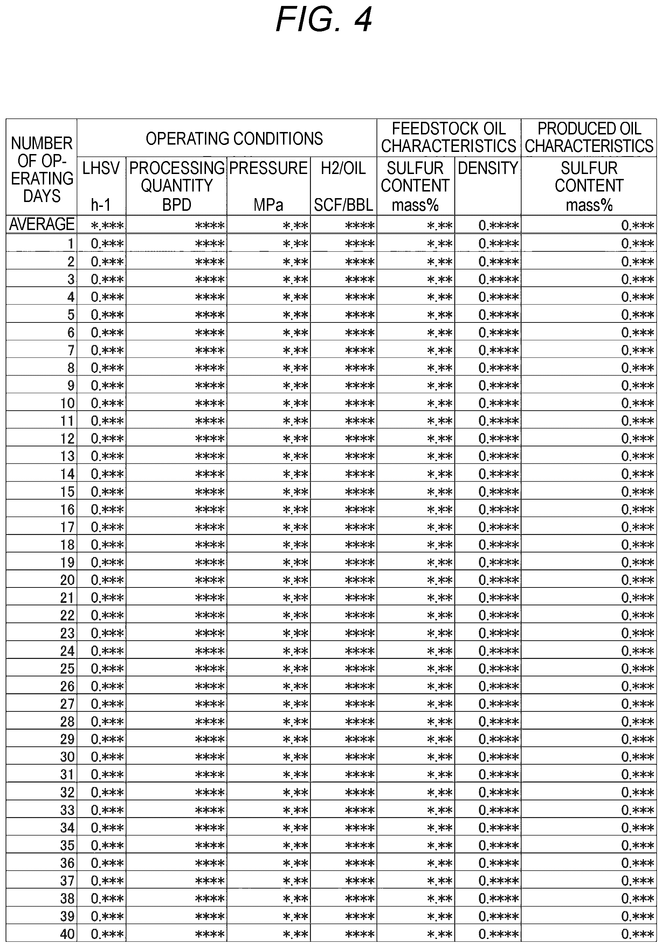

First, the user inputs user desulfurization catalyst performance prediction conditions from the user terminal 16 (S21), as shown in Fig. 4. In the present description, the "user desulfurization catalyst performance prediction conditions" are preconditions for predicting performance of the desulfurization catalyst in the user plant, and contain at least one of operating conditions, feedstock oil characteristics and produced oil characteristics.

The server computer 12 calculates the desulfurization catalyst-related information based on the input user desulfurization catalyst performance prediction conditions and the user desulfurization catalyst-related function stored in the user plant information memory 22 (S22).

For example, when the user desulfurization catalyst performance prediction conditions are applied to the user desulfurization catalyst lifetime function which has been tuned to the user plant use, a transition of required temperature of the desulfurization catalyst, i.e. a lifetime of the desulfurization catalyst can be calculated. The "required temperature" means reaction temperature necessary for obtaining a fixed sulfur content of refined oil.

When the user desulfurization catalyst performance prediction conditions are applied to a user hydrogen consumption function in a similar manner or the same manner, hydrogen consumption in the user plant can be calculated. When the user desulfurization catalyst performance prediction conditions are applied to a user product yield function, a product yield in the user plant can be calculated.

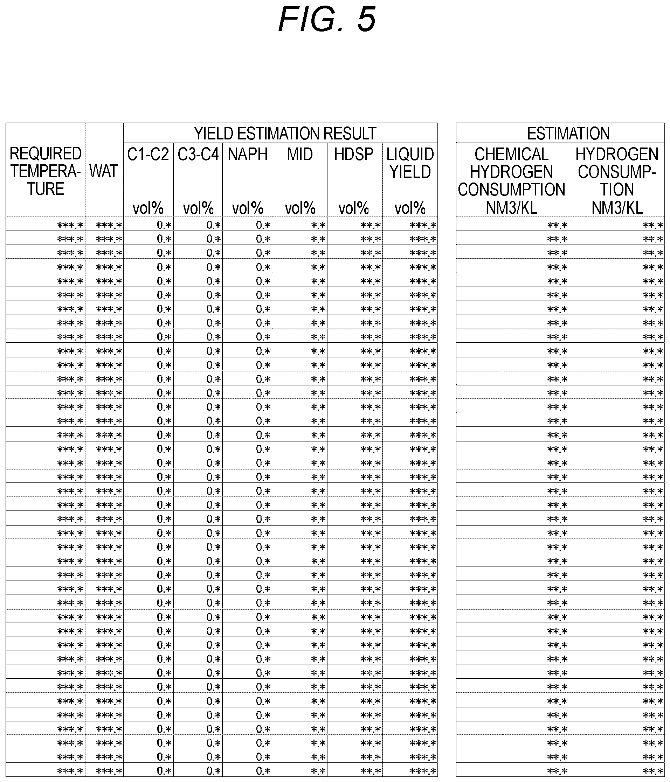

The desulfurization catalyst-related information calculated thus is transmitted from the server computer 12 to the communication server computer 14. The communication server computer 14 transmits the desulfurization catalyst-related information to the user terminal 16 via the network 18 (S23), and displays the desulfurization catalyst-related information shown in Fig. 5 on the user terminal 16.

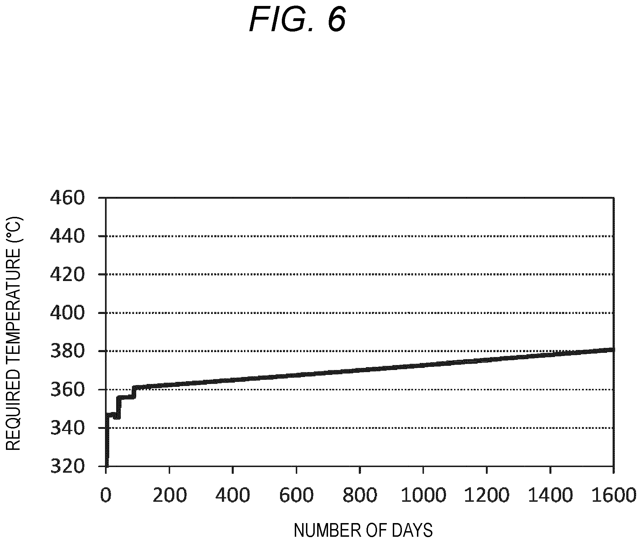

The communication server computer 14 may cause the user terminal 16 to perform display using an infographic, such as a graph indicating the number of lapsed days and the transition of the required temperature as the catalyst lifetime of the desulfurization catalyst, as shown in Fig. 6.

The desulfurization catalyst-related information includes at least the catalyst lifetime of the desulfurization catalyst, and may include the hydrogen consumption and the product yield. The desulfurization catalyst-related information may also include, for example, information about evaluation of economic efficiency, information about catalyst selection, etc.

The information about the evaluation of the economic efficiency can, for example, include manufacturing cost etc. of the produced oil calculated based on the catalyst lifetime of the desulfurization catalyst, a unit price of the feedstock oil, etc. The information about the catalyst selection can, for example, include information of a catalyst which can minimize the manufacturing cost of the produced oil, comparation information between the catalyst currently used by the user and the catalyst which can minimize the manufacturing cost, etc. by use of desulfurization catalyst pilot plant data of desulfurization catalysts stored in the desulfurization catalyst pilot plant data memory 20.

The desulfurization catalyst-related information is uniquely determined from the user plant-related data and the user desulfurization catalyst performance prediction condition. For example, where user plant-related data, a user desulfurization catalyst characteristic prediction condition, a user desulfurization catalyst-related function and desulfurization catalyst-related information of a user A are designated by A1, A2, fa, and A3 respectively, and user plant-related data, a user desulfurization catalyst characteristic prediction condition, a user desulfurization catalyst-related function and desulfurization catalyst-related information of a user B are designated by B1, B2, fb, and B3 respectively,
(1) if A1≠B1 and A2≠B2, then fa≠fb and A3≠B3
(2) if A1=B1 and A2≠B2, then fa=fb and A3≠B3
(3) if A1≠B1 and A2=B2, then fa#fb and A3≠B3
(4) if A1=B1 and A2=B2, then fa=fb and A3=B3

In the present example, the desulfurization catalyst pilot plant data stored in the desulfurization catalyst pilot plant data memory 20 is updated suitably by an administrator or the like of the desulfurization catalyst performance prediction system 10.

For example, after a lapse of one month, data about a predetermined catalyst α is accumulated. Therefore, when the desulfurization catalyst pilot plant data updated after one month is used, a lifetime can be predicted with higher accuracy than when the data of one month ago is used. Therefore, the same result as at present cannot be always obtained one month later even when the same values are input as the user plant-related data and the user desulfurization catalyst performance prediction conditions.

When the desulfurization catalyst-related information is intended to be obtained by use of the desulfurization pilot plant data updated suitably, it is rather general that a result which will be obtained one month later is different from a result which is obtained at present. Thus, the desulfurization catalyst-related information can be obtained by use of the desulfurization catalyst pilot plant data updated suitably. Accordingly, according to the desulfurization catalyst performance prediction system 10 of the present example, prediction can be made with higher accuracy as days go on.

The desulfurization catalyst-related information providing method can be carried out by the aforementioned procedure. When the desulfurization catalyst-related information is provided thus, the user can easily obtain information about a lifetime of the desulfurization catalyst with high accuracy even if the user does not possess the pilot plant etc.

While a preferred embodiment of the present invention has been described above, the present invention is not limited thereto. In the aforementioned example, the desulfurization catalyst performance prediction system 10 has been described as a client server model by use of the server computer 12. However, various changes can be made without departing from the object of the present invention. For example, the desulfurization catalyst performance prediction system 10 can be configured by use of a stand-alone computer.

The programs for making the computer execute generation of the aforementioned user desulfurization catalyst-related function and calculation of the desulfurization catalyst-related information and a computer-readable recording medium having the programs recorded thereon, such as a magnetic tape (such as a digital data storage (DSS)), a magnetic disk (such as a hard disk drive (HDD) or a flexible disk (FD)), an optical disc (such as a compact disc (CD), a digital versatile disc (DVD) or a Blu-ray disc (BD)), a magneto-optical disk (MO) or a flash memory (such as an SSD (Solid State Drive), a memory card or a USB memory) are also included as a form of the present invention.

The present application is based on a Japanese Patent Application No. 2016-247762 filed on December 21, 2016, the content of which is incorporated herein by reference.

### Industrial Applicability

According to the present invention, a performance of a desulfurization catalyst can be predicted with high accuracy without performing a complicated process such as a plant simulation.

### Description of Reference Signs

- 10: desulfurization catalyst performance prediction system
- 12: server computer
- 13: processor
- 14: communication server computer
- 16: user terminal
- 18: network
- 20: desulfurization catalyst pilot plant data memory
- 22: user plant information memory
- 24: desulfurization catalyst-related function memory
- 26: user information memory

## Claims

1. A server to be connected to a user terminal via a network to supply a desulfurization catalyst lifetime of a user plant to a user based on desulfurization catalyst pilot plant data and a desulfurization catalyst lifetime function, the server comprising:
a processor and a memory storing computer-readable instructions, wherein, when the computer-readable instructions are executed by the processor, the server
receives user plant-related data and a user desulfurization catalyst performance prediction condition from the user terminal,
generates from the desulfurization catalyst lifetime function a user desulfurization catalyst lifetime function tailored to the user plant based on a comparison between the desulfurization catalyst pilot plant data and the received user plant-related data,
calculates a catalyst lifetime for a user desulfurization catalyst based on the received user desulfurization catalyst performance prediction condition and the user desulfurization catalyst lifetime function, and
transmits the calculated catalyst lifetime to the user terminal.

2. The server according to claim 1, wherein, when the computer-readable instructions are executed by the processor, the server further
generates from a hydrogen consumption function a user hydrogen consumption function tailored to the user plant based on the comparison between the desulfurization catalyst pilot plant data and the received user plant-related data,
calculates a hydrogen consumption for the user desulfurization catalyst based on the received user desulfurization catalyst performance prediction condition and the user hydrogen consumption function, and
transmits the calculated hydrogen consumption to the user terminal.

3. The server according claim 1 or 2, wherein, when the computer-readable instructions are executed by the processor, the server further
generates from a product yield function a user product yield function tailored to the user plant based on the comparison between the desulfurization catalyst pilot plant data and the received user plant-related data,
calculates a product yield for the user desulfurization catalyst based on the received user desulfurization catalyst performance prediction condition and the user product yield function, and
transmits the calculated product yield to the user terminal.

4. The server according to any one of claims 1 to 3, wherein the server calculates a tuning parameter based on a comparison between the user plant-related data and the desulfurization catalyst pilot plant data, and
generates, from the tuning parameter and the desulfurization catalyst-related function, the user desulfurization catalyst lifetime function tailored to the user plant.

5. The server according to any one of claims 1 to 4, wherein the user plant-related data includes at least one of
user plant operating data, the user plant operating data being operating data for a predetermined period in the user plant and including at least an operating condition, a feedstock oil characteristic and a produced oil characteristic,
a kind of catalyst to be used in the user plant, and
equipment setting information of the user plant.

6. The server according to any one of claims 1 to 5, wherein the user desulfurization catalyst performance prediction condition includes at least one of an operating condition, a feedstock oil characteristic and a produced oil characteristic.

7. The server according to any one of claims 1 to 6, wherein the desulfurization catalyst lifetime function includes at least one of a degradation function for direct desulfurization, a degradation function for indirect desulfurization, a degradation function for light gas oil desulfurization, and a degradation function for kerosene desulfurization.

8. The server according to any one of claims 1 to 7, wherein the server receives the user plant-related data including at least a kind of catalyst, and
generate the user desulfurization catalyst lifetime function based on the desulfurization catalyst pilot plant data corresponding to the kind of catalyst.

9. A method to be executed by a processor, for supplying a desulfurization catalyst lifetime of a user plant to a user via a network based on desulfurization catalyst pilot plant data and a desulfurization catalyst lifetime function, the method comprising steps of:
receiving user plant-related data and a user desulfurization catalyst performance prediction condition from the user terminal;
generating from the desulfurization catalyst lifetime function a user desulfurization catalyst lifetime function tailored to the user plant based on a comparison between the desulfurization catalyst pilot plant data and the received user plant-related data;
calculating a catalyst lifetime for a user desulfurization catalyst based on the received user desulfurization catalyst performance prediction condition and the user desulfurization catalyst lifetime function; and
transmitting the calculated catalyst lifetime information to the user terminal.

10. The method according to claim 9, comprising steps of:
generating, from a hydrogen consumption function, a user hydrogen consumption function tailored to the user plant based on the comparison between the desulfurization catalyst pilot plant data and the received user plant-related data;
calculating a hydrogen consumption for the user desulfurization catalyst based on the received user desulfurization catalyst performance prediction condition and the user hydrogen consumption function; and
transmitting the calculated hydrogen consumption to the user terminal.

11. The method according to claim 9 or 10, comprising:
generating, from a product yield function, a user product yield function tailored to the user plant based on the comparison between the desulfurization catalyst pilot plant data and the received user plant-related data;
calculating a product yield for the user desulfurization catalyst based on the received user desulfurization catalyst performance prediction condition and the user product yield function; and
transmitting the calculated product yield to the user terminal.

12. The method according to any one of claims 9 to 11, comprising steps of:
calculating a tuning parameter based on a comparison between the user plant-related data and the desulfurization catalyst pilot plant data; and
generating, from the tuning parameter and the user desulfurization catalyst lifetime function, the user desulfurization catalyst lifetime function tailored to the user plant.

13. The method according to any one of claims 9 to 12, wherein the user plant-related data includes at least one of:
user plant operating data, the user plant operating data being operating data for a predetermined period in the user plant and including at least an operating condition, a feedstock oil characteristic and a produced oil characteristic;
a kind of catalyst to be used in the user plant; and
equipment setting information of the user plant.

14. The method according to any one of claims 9 to 13, wherein the desulfurization catalyst performance prediction condition includes at least one of an operating condition, a feedstock oil characteristic and a produced oil characteristic.

15. The method according to any one o claims 9 to 14, wherein the desulfurization catalyst lifetime function includes at least one of a degradation function for direct desulfurization, a degradation function for indirect desulfurization, a degradation function for light gas oil desulfurization, and a degradation function for kerosene desulfurization.

16. The method according to any one of claims 9 to 15, comprising receiving the user plant-related data including at least a kind of catalyst; and
generating the user desulfurization catalyst lifetime function based on the desulfurization catalyst pilot plant data corresponding to the kind of catalyst.

17. A program for causing a computer to execute the method according to any one of claims 9 to 16.

18. A computer-readable recording medium storing the program according to claim 17.
